# EUROPEAN PATENT APPLICATION

(11) **EP 4 176 802 A1**
(43) Date of publication of application: **10.05.2023**
(21) Application number: 21206112.1
(22) Date of filing: 03.11.2021
(51) Int. Cl.: A61B 5/0205, A61B 5/00, G16H 40/60, G06F 3/048

(54) **SYSTEM AND METHOD FOR PATIENT MONITORING**

(71) Applicant: Koninklijke Philips N.V., 5656 AG Eindhoven (NL)
(72) Inventor: LOPEZ LOPEZ, Benjamin, Eindhoven (NL); HUIJBREGTS, Laurentia Johanna, Eindhoven (NL); BULUT, Murtaza, Eindhoven (NL); VAN LIESHOUT, Ron Martinus Laurentius, Eindhoven (NL)
(74) Representative: Philips Intellectual Property & Standards

(57) **Abstract**

A system and method are provided for patient monitoring. The system and method may receive measurement data comprising measured values of physiological parameters of a patient at consecutive time instances. On a display, a trend view 510 may be provided which provides a longitudinal visualization of the measurement data by setting out the measured values of a first set of the physiological parameters at the consecutive time instances against a common timeline. Simultaneously with the trend view, a patient status view 500 may be provided which provides a visualization of a physiological state of the patient and which visualization is adapted to the measured values of a second set of the physiological parameters at a select time instance. The trend view and the patient status view may be dynamically linked by, when user input is received which is indicative of a selection of a past time instance, adapting the visualization of the physiological state to the measurements of the second set physiological parameters at the past time instance.

## Description

### FIELD OF THE INVENTION

The invention relates to a system and computer-implemented method for patient monitoring. The invention further relates to a computer-readable medium comprising instructions to perform the computer-implemented method.

### BACKGROUND OF THE INVENTION

It is known to monitor physiological parameters of a subject at regular and consecutive time instances.. If the monitoring is in the context of medical care, the subject may also be referred to as patient and such monitoring as patient monitoring. In both cases, the measured values of the physiological parameters may be visualized, for example for the user or patient to see (e.g., in self-care scenarios) or for medical personnel to see. Typically, in patient monitoring, the measured values may be compared to static or dynamic thresholds so detect abnormalities in the physical parameters, and if such abnormalities are detected, an alarm may be triggered, e.g., to attract the attention of medical personnel. For example, on an intensive care unit (ICU), a patient's vital signs (heart rate (HR), respiration rate (RR), core body temperature (CBT), oxygen saturation (SpO2) and blood pressure (BP)) may be closely monitored.

However, in some cases, it may not suffice to monitor only the regular vital signs, and advanced hemodynamic measurements may be needed to keep a better eye on the patient, for example if the patient has hemodynamic complications and/or to diagnose the cause of these complications. Advanced hemodynamic parameters to be measured may include cardiac output (CO), cardiac index (CI), stroke volume (SV), stroke volume index (SVI), stroke volume variation (SVV), ejection fraction (EF), systemic vascular resistance (SVR), systemic vascular resistance index (SVRI), pulmonary vascular resistance (PVR), pulmonary vascular resistance index (PVRI), pulmonary artery pressure (PAP), pulmonary artery wedge pressure (PAWP), extravascular lung water index (ELWI) and global end-diastolic volume index (GEDVI). There are various modalities available to measure advanced hemodynamic parameters, varying in which parameters they measure, their invasiveness, and their accuracy. Examples of such modalities include, but are not limited to, a Swan Ganz pulmonary artery catheter (PAC), Philips PiCCO (which combines an arterial line and a central venous line), Edwards ClearSight (which uses finger cuffs), Edwards FloTrac (using an arterial line), echography, and the Philips double-cuff system.

Although intensivists can interpret the values of such advanced hemodynamic parameters, other medical personnel, such as physician assistants, residents, and nurses are typically less familiar with such hemodynamic parameters. To help them interpret the measurement values, a visualization of a physiological state of the patient may be provided, which visualization of the physiological state may be adapted to the measured values of the advanced hemodynamic parameters. Such adaptation may involve adjusting a visual attribute of one or more visual elements of the visualization so as to provide a visual representation of the measured values of the advanced hemodynamic parameters.

For example, a visualization of a physiological state is described in co-pending application EP EP21150858.5 of the applicant, which describes a figure-eight schematic that represents a circulatory system of the patient. A first loop of the figure-eight schematic may represent pulmonary circulation of the patient. A second loop of the figure-eight schematic, which in some embodiments may be presented underneath the first loop, may represent systemic circulation of the patient. A central rounded object that connects the first and second loops may represent a heart of the patient, and in some embodiments may be shaped to convey end diastolic volume (EDV) of the patient. One or both of the first and second loops may include multiple arc fragments. Each arc fragment may be shaped to convey one or more of the measured physiological parameters of the patient. For example, various arc fragments can have relative widths or thicknesses that convey various medical conditions depending on which arc fragments are thicker and which are thinner.

Another type of visualization of a physiological state is a Frank-Starling curve [3], which on the horizontal axis provides a measure of ventricular preload and is usually either left ventricular end-diastolic volume (LVEDV) or left ventricular end-diastolic pressure (LVEDP), while the vertical axis gives a measure of the force of contraction of the heart and is usually stroke volume (SV) but may also be cardiac output (CO). This plot may visually convey the condition of the heart of a particular patient.

Yet another example is a so-called "visual patient" [1], [2], being an animated avatar, which changes its appearance to reflect values of various physiological parameters. For example, the skin color of the avatar may be adapted to the oxygen saturation (SpO2), for example to visualize low oxygen saturation by a cyanotic skin color. Similarly, pulse and respiratory frequency may be shown by animating the avatar. While the visual patient of [1], [2] is described to visualize regular vital signs, such types of avatars may also be used to provide visualizations of advanced hemodynamic parameters of a patient.

In general, the physiological parameters to be measured may include one or more of advanced hemodynamic parameters, regular vital signs, concentrations of biomarkers, hormones, neurochemicals, electrolytes in blood, in sweat or in expired air, etc. In general, the measured values of physiological parameters may change over time, with some physiological parameters fluctuating within minutes or hours and others showing only considerable changes over days or months. Changes over time may be particularly relevant for medical personnel, e.g., to be able to see if a patient is getting better, is stable, or is deteriorating, or to see if certain interventions (medication/fluids) have an effect.

Disadvantageously, the visualizations of the physiological state of the patient, such as those identified above, often already extend in all available spatial dimensions (typically in two-dimensions (2D) in case the visualization is shown on a display), for example to show the avatar of the patient or a visual representation of a circulatory system of the patient. This means that there may not be space to visualize a time dimension, and that the visualization may need to be limited to a particular time instance, e.g., to visualize only current measurement values. Thereby, such visualizations of the physiological state of a patient may lack interpretability for physician assistants, residents, and nurses and may fail to show whether a patient is getting better or worse or whether interventions have an effect.

### References

[1] "User perceptions of avatar-based patient monitoring: a mixed qualitative and quantitative study", David W. Tscholl et al., BMC Anesthesiology (2018) 18:188
[2] "Avatar-based patient monitoring in critical anaesthesia events: a randomized high-fidelity simulation study", Tadzio R. Roche et al., Quality and Patient Safety, Vol. 126(5), P1046-1054, May 01, 2021
[3] Anaesthesia Key, chapter 30: Starling's Law and Cardiac Dysfunction https://aneskey.com/chapter-30-starlings-law-and-cardiac-dysfunction/

### SUMMARY OF THE INVENTION

It may be desirable to obtain a system and computer-implemented method for patient monitoring which provides a visualization of a physiological state of the patient which allows a user to obtain insights into longitudinal changes in the physiological parameters.

In accordance with a first aspect of the invention, a system for patient monitoring is provided, comprising:
an input interface for receiving measurement data comprising measured values of physiological parameters of a patient at consecutive time instances;
a user interface subsystem comprising:
   - a user input interface to a user input device for receiving user input;
   - a display output to a display for displaying output of the system;
   a processor subsystem configured to, via the user interface subsystem:
   - provide a trend view which provides a longitudinal visualization of the measurement data by setting out the measured values of a first set of the physiological parameters at the consecutive time instances against a common timeline;
   - provide a patient status view in separation of the trend view, wherein the patient status view provides a visualization of a physiological state of the patient at a select time instance, wherein the visualization of the physiological state is adapted to the measured values of a second set of the physiological parameters at the select time instance; and
   - dynamically link the trend view and the patient status view by:
receiving a selection of a past time instance;
in response, adapting the visualization of the physiological state to the measurements of the second set physiological parameters at the past time instance.

In accordance with a further aspect of the invention, a computer-implemented method for patient monitoring is provided, comprising:
receiving measurement data comprising measured values of physiological parameters of a patient at consecutive time instances;
on a display, providing a trend view which provides a longitudinal visualization of the measurement data by setting out the measured values of a first set of the physiological parameters at the consecutive time instances against a common timeline;
on the display, providing a patient status view in separation of the trend view, wherein said providing of the patient status view comprises providing a visualization of a physiological state of the patient at a select time instance, wherein the visualization is adapted to the measured values of a second set of the physiological parameters at the select time instance; and
dynamically linking the trend view and the patient status view by:
   - receiving a selection of a past time instance;
   - in response, adapting the visualization of the physiological state to the measurements of the second set physiological parameters at the past time instance.

In accordance with yet a further aspect of the invention, a transitory or non-transitory computer-readable medium is provided which medium comprises data representing a computer program, the computer program comprising instructions for causing a processor system to perform the above-identified computer-implemented method.

The above measures relate to patient monitoring, which may typically involve monitoring the condition of a patient in a clinical setting, e.g., in a hospital, but which may also include non-clinical settings, e.g., at home in a self-care setting, as well as non-medical settings, e.g., when monitoring the performance of a sporter. In such patient monitoring, physiological parameters of the patient may be measured at consecutive time instances, for example at regular intervals, e.g., every 100ms, 1 sec, 1 minute, 10 minutes, 1 hour, etc., or at irregular intervals, e.g., when changes in the physiological parameters are expected to occur. For such measurements, various known types of sensors and sensing devices may be used, as for example described in the background section of this specification.

The system and method are configured to obtain measurement data of such physiological parameters and to provide a visualization of a physiological state of the patient based on the measurement data of a set of these physiological parameters. This visualization is also referred to as 'patient status view' and may provide a physiological or anatomical interpretation of the measured values of the physiological parameters. For example, the visualization may translate measured values into visual attributes of visual elements so that the visual elements represent the measured values in a physiologically or anatomically meaningful manner. In a specific and concrete example, a low numerical value of a temperature measurement may be translated into a bluish or purplish skin color of an avatar so as to provide a physiological interpretation of the low numerical value. Effectively, the visualization may provide a higher-level view of the physiological state of the patient than the mere numerical measurement values. In some examples, the patient status view may even omit the display of numerical values, or may merely display the numerical values as an augmentation of the visualization of a physiological state of the patient. Such types of visualizations are known per se, as shown by the examples in the background section.

As also elucidated in the background section, the patient status view may provide a visualization of the physiological state of the patient at one time instance as it may not allow for the visualization of the physiological state at several time instances simultaneously. The patient status view may thus lack a temporal dimension or time axis. Nevertheless, a clinician may wish to see the patient's physiological state at a number of time instances, for example in the past, for example to be able to determine what lead to the patient's current situation or to specifically investigate events which occurred in the past.

The above measures therefore provide a separate longitudinal visualization of the measurement data in which the measured values of a second set of the physiological parameters are set out against a common timeline. Here, 'common' may refer to the timeline applying to each of the visualized physiological parameters. As also discussed elsewhere, this second set of physiological parameters may be the same as that which is visualized in the patient status view, but may also be different. The longitudinal visualization may for example comprise, for each of the set's physiological parameters, a graph plotting the measurement values along the y-axis and the time along the x-axis. Such a longitudinal visualization may allow a user to obtain a temporal overview and to identify trends in the physiological parameters. For that reason, the longitudinal visualization may elsewhere also be referred to as 'trend view'. The above measures then dynamically link the trend view and the patient status view by receiving a selection of a past time instance, and in response, adapting the patient status view to the measurements of the second set of physiological parameters at the past time instance. Typically, the selection of the past time instance may be received as user input, but in some embodiments may also represent an automatic selection by the system as claimed. Here, the term 'link' may refer to the selection of the past time instance taking place in the trend view or at least as a result of having viewed the trend view, which selection then causes the patient status view to be adapted. Moreover, such linking may be 'dynamic' in that it may be responsive to a user's selection and cause the patient status view to be dynamically adapted to the selection.

The above measures allow a user to obtain a faster comprehensive understanding of the patient's physiological parameters by providing a longitudinal visualization, which a user may use as a basis to select a time instance and which then results in the more interpretative patient status view being shown for this time instance. This type of graphical user interface has been clinically evaluated and was found to greatly help clinicians in understanding more complex type of physiological parameters and to prevent tunnel vision. For example, it has been found that for advanced hemodynamic parameters, the trend view itself may be difficult to interpret for clinicians other than intensivists, while the patient status view may be easier to interpret as itself already provides an interpretation of these advanced hemodynamic parameters. By showing both views simultaneously and dynamically linking both views, a clinician may effectively 'navigate' in time through hemodynamic trends in the trend view, with the patient status view at these time instances helping a clinician to obtain an understanding of the patient's physiological situation at the respective time instance. As a result, a clinician may quicker obtain an overview of the patient's situation and, if needed, take clinical action. In addition, staff which may otherwise have difficulty to interpret hemodynamic monitoring values may be enabled to use and interpret these hemodynamic monitoring values through the provided visualizations.

In some examples, the patient status visualization may show a frame of a video, such as an ultrasound video, and the user may seek through the video by selecting a time instance on the common timeline of the trend view. In such examples, the visualization of the physiological status of the patient may thus be represented by a video of the patient.

Optionally, the visualization of the physiological state of the patient comprises one or more visual elements, wherein the visualization is adapted to the measured values of the second set of the physiological parameters by adjusting a visual attribute of at least one of the one or more visual elements. The visualization of the physiological state of the patient may be comprised of visual elements, such as geometric primitives (e.g., lines or polygons) or 2D/3D computer graphics objects (e.g., comprised of vertices, edges and faces), which together may provide the visualization of the physiological state. Such visual elements may, either individually or jointly with other visual elements, convey measurement values through their visual appearance. For example, one or more visual elements representing an avatar's legs may by way of their shape convey whether the patient has a high or low neuromuscular activity (with limb legs indicating low activity). In general, the one or more visual elements may visually represent one or more anatomical or physiological attributes of the patient. The visual attribute may be one of: a spatial attribute such as a position, orientation, size or shape, a color, a transparency, and a pattern, of the visual element.

Optionally, the patient status view comprises an avatar or a visual representation of a physiological system of the patient. The term 'avatar' may refer to a visual representation of the patient, which may, but does need to be, personalized to the specific patient, e.g., in terms of sex or general physical appearance. Examples of physiological systems include the circulatory system or the respiratory system.

Optionally, the processor subsystem is configured to, via the user interface subsystem, enable a user to provide the user input indicative of the selection of the past time instance by specifying a point on the common timeline. A user may wish to select a time instance for the patient status view based on the trend view, in that he/she may identify a time instance of interest in the trend view. By allowing a user to specify a point on the common timeline in the trend view, the user may easily select this time instance of interest.

Optionally, the processor subsystem is configured to enable the user to specify the point on the common timeline by at least one of:
- moving a visual element onscreen relative to a visualization of the common timeline in the trend view;
- entering a numerical value, and
- selecting a visual element which modifies a currently selected time instance.

In general, such and other type of user input may be provided in various known ways, e.g., using touch screen, a mouse, voice recognition, gesture recognition, etc.

Optionally, the processor subsystem is configured to, via the user interface subsystem, animate the visualization of the physiological state of the patient shown in the patient status view by sequentially adapting the visualization of the physiological state to the measured values of the second set of the physiological parameters at the consecutive time instances and by using the selected time instance as start-time or end-time of said animation. In case the visualization of the physiological state is comprised of visual elements such as geometric primitives or 2D/3D computer graphics objects, the visualization may be animated to show the physiological state of the patient at consecutive time instances. This may allow a user to see a trend or other type of development of the physiological status.

Optionally, the processor subsystem is configured to provide, via the user interface subsystem, playback controls to enable the user to control said animation of the visualization of the physiological state, wherein the playback controls comprise at least one of: a pause function, a resume playback function, a forward function, a reverse function, a loop selection function, and a playback speed adjustment function. This way, a user may control the playback of the animation of the visualization of the physiological state. In some examples, said visualization may be or comprise a video. In such examples, the playback controls may be used by the user to control the playback of the video, e.g., its speed. In addition or alternatively to enabling a user to control the playback, the playback may also be controlled automatically. For example, the playback speed may be controlled by the processor subsystem to increase the playback speed when there are fewer changes in the values of the physiological parameters and to decrease the playback speed if there are more changes. For example, the processor subsystem may quantify the amount of changes in a given time period and determine the playback speed based on said quantified amount.

Optionally, the processor subsystem is configured to enable, via the user interface subsystem, the user to select a physiological parameter in the trend view to, in the patient status view, enable or disable adapting the visualization of the physiological state to the select physiological parameter, or to highlight said adaptation in the visualization of the physiological state.

Optionally, the processor subsystem is configured to enable, via the user interface subsystem, a user to set an alarm threshold for at least one of the physiological parameters in one of: the trend view and the patient status view, wherein the processor subsystem is configured to visualize the alarm threshold in the other one of: the trend view and the patient status view.

Optionally, the first set and the second set of the physiological parameters are identical or different by one of said sets being a subset of, or being derived from, the other set of the physiological parameters.

Optionally, the physiological parameters comprise one or more of: cardiac output (CO), cardiac index (CI), stroke volume (SV), stroke volume index (SVI), stroke volume variation (SVV), ejection fraction (EF), systemic vascular resistance (SVR), systemic vascular resistance index (SVRI), pulmonary vascular resistance (PVR), pulmonary vascular resistance index (PVRI), pulmonary artery pressure (PAP), pulmonary artery wedge pressure (PAWP), extravascular lung water index (ELWI) and global end-diastolic volume index (GEDVI).

It will be appreciated by those skilled in the art that two or more of the above-mentioned embodiments, implementations, and/or optional aspects of the invention may be combined in any way deemed useful.

Modifications and variations of the system, the computer-implemented method and/or the computer program product, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 shows a system for patient monitoring, which system comprises an input interface for receiving measurement data of physiological parameters of a patient and a user interface subsystem for displaying a graphical user interface on a display to a user;
Figs. 2A and 2B show a visualization of a physiological state of a patient in the form of a figure-eight schematic which represents a circulatory system of the patient, and which may be adapted to reflect measured values of physiological parameters of the patient;
Fig. 3 shows a visualization of a physiological state of a patient in the form of an avatar which changes its appearance to reflect measured values of physiological parameters of the patient;
Fig. 4 shows a longitudinal visualization of measurement data of a patient, which sets out measured values of physiological parameters against a common timeline;
Fig. 5 shows another type of longitudinal visualization of measurement data;
Fig. 6 shows an example of display output of the system of Fig. 1, simultaneously showing a visualization of a physiological state of a patient and a longitudinal visualization of measurement data of the patient, with both visualizations being dynamically linked in that a user may select a time instance in the longitudinal visualization, causing the system to adapt the visualization of the physiological state of the patient to the measured values of physiological parameters at the selected time instance;
Fig. 7 is similar to Fig. 6 but shows another type of longitudinal visualization;
Fig. 8 is similar to Fig. 6 but shows another type of visualization of the physiological state of the patient and another type of longitudinal visualization;
Fig. 9 is similar to Fig. 8 but shows another way of selecting a time instance;
Fig. 10 shows a method for patient monitoring; and
Fig. 11 shows a non-transitory computer-readable medium comprising data.

It should be noted that the figures are purely diagrammatic and not drawn to scale. In the figures, elements which correspond to elements already described may have the same reference numerals.

### List of reference numbers

The following list of reference numbers is provided for facilitating the interpretation of the drawings and shall not be construed as limiting the claims.
- 20: data storage
- 40: physiological signals
- 42: measurement data
- 60: display
- 62: display data
- 80: user input device
- 82: user input data

- 100: system for patient monitoring
- 110: data storage interface
- 120: sensor interface
- 140: processor subsystem
- 142-148: data communication
- 160: memory
- 180: user interface subsystem
- 182: display output interface
- 184: user input interface

- 222: figure-eight schematic
- 224: first (top) loop
- 226: second (bottom) loop
- 228: central object
- 230: arc fragment
- 232: arc length
- 234: arc fragment
- 236: arc fragment envelope
- 238: arc-shaped channel visualizing SVR
- 239: inwardly-pointing arrows
- 240: arc fragment
- 241: arc fragment
- 242: arc fragment
- 243: double-headed arrow
- 244: arc fragment envelope
- 245: double-headed arrow
- 246: arc-shaped channel
- 247: inwardly-pointing arrows
- 300: avatar
- 310: visual element(s) representing floppy legs
- 320: visual element(s) representing closed eyes
- 330: visual element(s) representing low body temperature

- 400, 402: trend view
- 410: time axis
- 420: slider for selecting time instance
- 430: physiological parameter
- 440, 442: longitudinal visualization of measurement values

- 500: figure-eight schematic adapted to select time instance
- 510, 512: trend view
- 520: slider for selecting time instance

- 600: avatar adapted to select time instance
- 610: trend view
- 620: slider for selecting time instance
- 630: numerical input field to enter time instance

- 700: method of patient monitoring
- 710: receiving measurement data
- 720: providing trend view
- 730: providing patient status view
- 740: dynamically linking trend view and patient status view

- 800: non-transitory computer-readable medium
- 810: data representing computer program

### DETAILED DESCRIPTION OF EMBODIMENTS

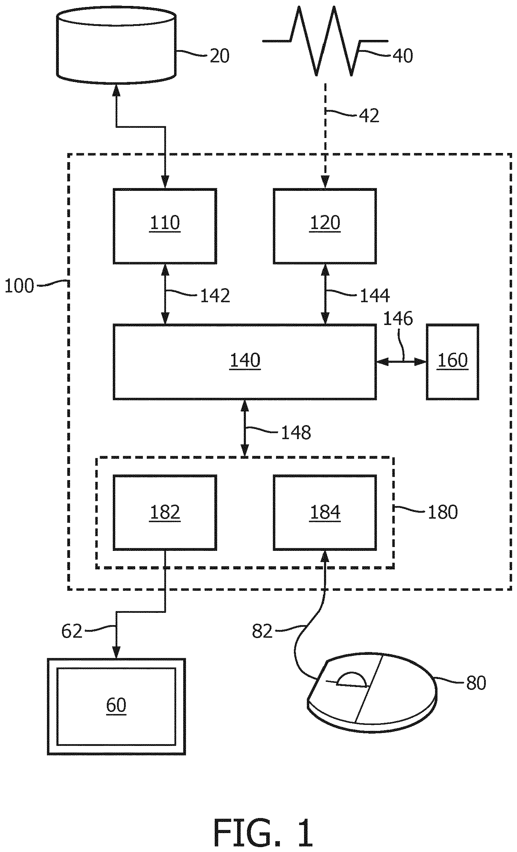

Fig. 1 shows a system 100 for patient monitoring. The system 100 may comprise a sensor interface 120 for obtaining measurement data 42 representing measurement values of physiological signals 40. Such measurement data 42 may for example be obtained from a sensor or from a sensing device, or a combination of such sensors and/or sensing devices. Examples of sensors and sensing devices include, but are not limited to, a Swan Ganz pulmonary artery catheter (PAC), Philips PiCCO (which combines an arterial line and a central venous line), Edwards ClearSight (which uses finger cuffs), Edwards FloTrac (using an arterial line), echography, Philips ShellCuff (using a cuff around the upper arm), etc. The measurement data may for example be received in real-time or near real-time. Effectively, the sensor interface 120 may constitute an input interface for the measurement data 42 and may elsewhere also be referred to as input interface.

The system 100 may further comprise a data storage interface 110 to a data storage 20. The data storage 20 may serve as short term and/or long-term data storage. For example, the measurement data 42 obtained via the sensor interface 120 may be at least temporarily stored on the data storage 20. In some embodiments, the measurement data 42 may also be accessed from the data storage 20 instead of using a sensor interface, for example in cases when the measurement data 42 is pre-recorded measurement data or when the measurement data 42 represents simulated measurement data. In the example of Fig. 1, the data storage interface 110 is shown to be connected to an external data storage 20. Alternatively, the data storage 20 may be an internal data storage of the system 100 (not shown in Fig. 1). In general, the data storage interface 110 may take various forms, such as a hard disk or solid-state disk interface to one or more hard disks and/or solid state disks, or a network interface to a Local Area Network (LAN) or a Wide Area Network (WAN).

The system 100 is further shown to comprise a processor subsystem 140 configured to internally communicate with the sensor interface 120 via data communication 144, with the data storage interface 110 via data communication 142, with a memory 160 via data communication 146 and with a user interface subsystem 180 via data communication 148. The memory 160 may for example be a volatile memory in which a computer program may be loaded which may cause the processor subsystem 140 to carry out functions which are described in this specification as being performed by the processor subsystem.

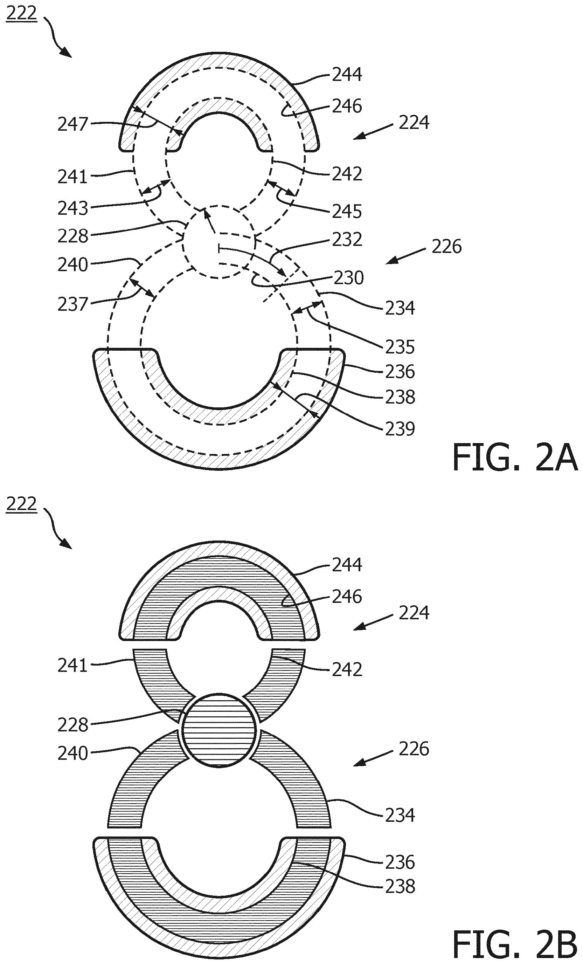

The user interface subsystem 180 may be configured to, during operation of the system 100, enable a user to interact with the system 100, for example using a graphical user interface. In particular, as also described with reference to Fig. 2 et seq., the graphical user interface may enable the user to select, or adjust a selection of, a time instance. For that and other purposes, the user interface subsystem 180 is shown to comprise a user input interface 184 configured to receive user input data 82 from a user input device 80 operable by the user. The user input device 80 may take various forms, including but not limited to a computer mouse, touch screen, keyboard, microphone, etc. Fig. 1 shows the user input device to be a computer mouse 80. In general, the user input interface 184 may be of a type which corresponds to the type of user input device 80, i.e., it may be a thereto corresponding type of user device interface. The user interface subsystem 180 is further shown to comprise a display output interface 182 configured to provide display data 62 to a display 60 to visualize output of the system 100. In the example of Fig. 1, the display is an external display 60. Alternatively, the display may be an internal display of the system 100.

As also described with reference to Fig. 2 et seq., the processor subsystem 140 may be configured to, during operation of the system 100 and using an input interface such as the sensor interface 120 or the data storage interface 110, receive measurement data 42 comprising measured values of physiological parameters of a patient at consecutive time instances, and using the user interface subsystem 180, provide a trend view which provides a longitudinal visualization of the measurement data by setting out the measured values of a first set of the physiological parameters at the consecutive time instances against a common timeline, provide a patient status view in separation of the trend view, wherein the patient status view provides a visualization of a physiological state of the patient at a select time instance, wherein the visualization of the physiological state is adapted to the measured values of a second set of the physiological parameters at the select time instance, and dynamically link the trend view and the patient status view by i) receiving user input indicative of a selection of a past time instance and ii) in response, adapting the visualization of the physiological state to the measurements of the second set physiological parameters at the past time instance. These and other operations of the system 100, and various optional aspects thereof, will be explained in more detail with reference to Fig. 2 et seq.

In general, the system 100 may be embodied as, or in, a single device or apparatus. The device or apparatus may be a general-purpose device or apparatus, such as a workstation or a computer, but may also be application-specific, such as a patient monitor. The device or apparatus may comprise one or more microprocessors which may represent the processor subsystem, and which may which execute appropriate software. The software may have been downloaded and/or stored in a corresponding memory, e.g., a volatile memory such as RAM or a non-volatile memory such as Flash. Alternatively, the functional units of the system, e.g., the input interface, the user interface subsystem, and the processor subsystem, may be implemented in the device or apparatus in the form of programmable logic, e.g., as a Field-Programmable Gate Array (FPGA). In general, each functional unit of the system 100 may be implemented in the form of a circuit. It is noted that the system 100 may also be implemented in a distributed manner, e.g., involving different devices or apparatuses. For example, the distribution may be in accordance with a client-server model, e.g., using a server and workstation. For example, the user input interface and the display output interface may be part of the workstation, while the processor subsystem may be a subsystem of the server. It is noted that various other distributions are equally conceivable.

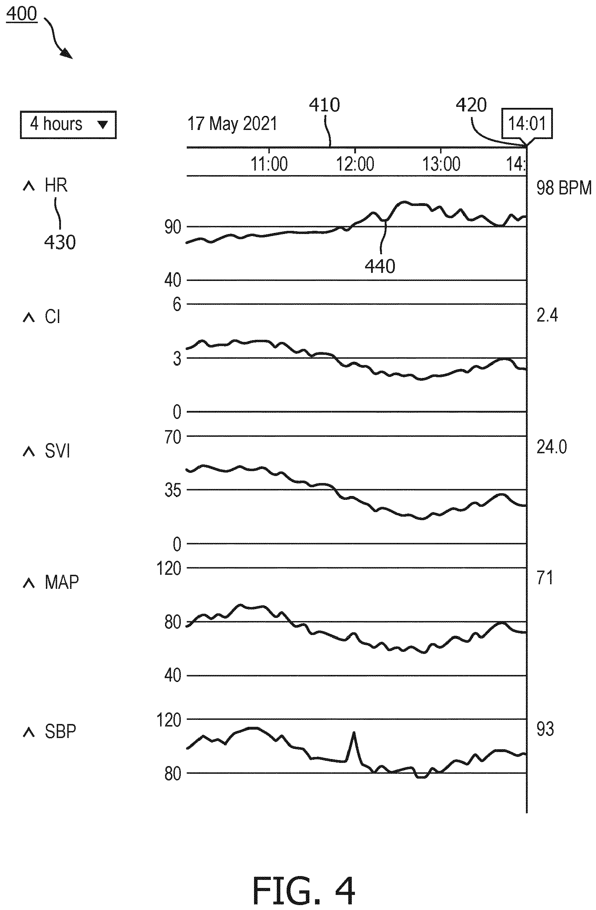

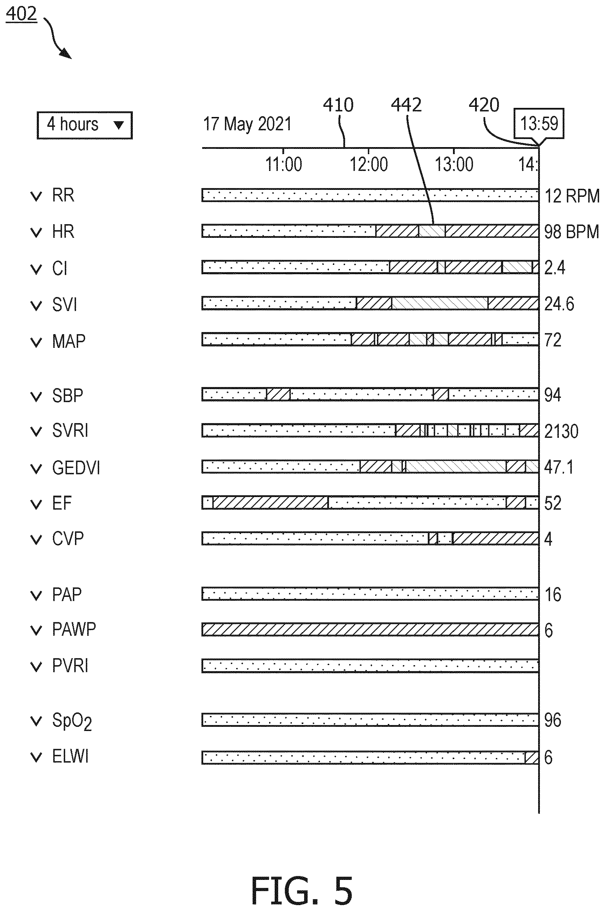

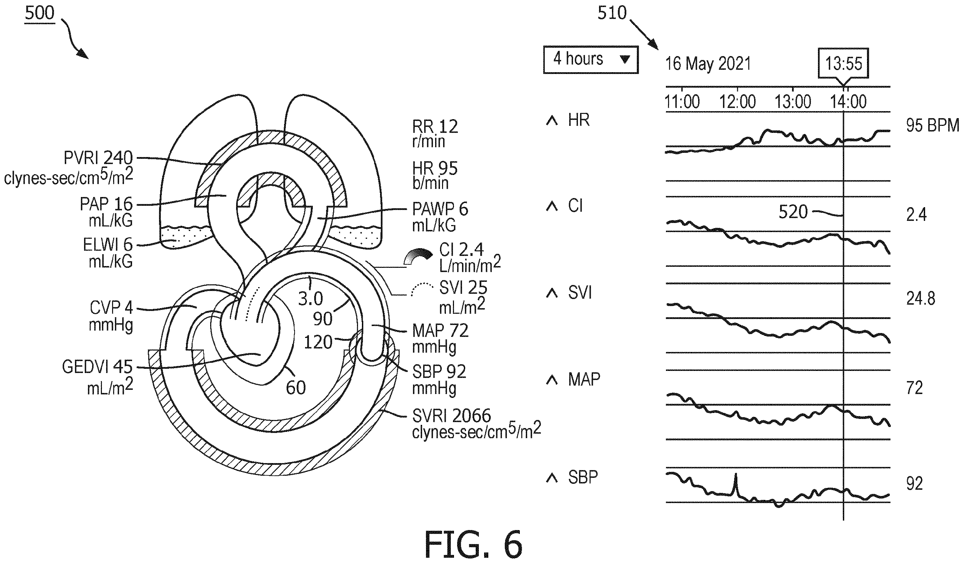

The following describes with reference to Figs. 2A-3 different visualizations of a physiological state of a patient, which visualizations are elsewhere also referred to as 'patient status views', with reference to Figs. 4, 5 different longitudinal visualizations of measurement data of a patient, which visualizations are elsewhere also referred to as 'trend views', and with reference to Figs. 6-9 different simultaneous visualizations of the patient status view and the trend view, which visualizations are dynamically linked to each other.

Figs. 2A, 2B show a visualization of a physiological state of a patient in the form of a figure-eight schematic which represents a circulatory system of the patient, and which may be adapted to reflect measured values of physiological parameters of the patient. This visualization is previously described in the co-pending application EP21150858.5 of the applicant, which is hereby incorporated by reference in as far as pertaining to the figure-eight schematic and its adaptation to the measured values of the physiological parameters. In particular, Figs. 2A and 2B depict an example figure-eight schematic 222 that may be rendered as part of a graphical user interface (GUI). Fig. 2A includes a number of visual annotations in the form of arrows that demonstrate how various portions can be shaped to convey various measured physiological values of a patient. Fig. 2B depicts in greater detail how figure-eight schematic may be divided logically into a plurality of arc fragments, each corresponding to a different measured physiological parameter of the patient.

Referring to both Figs. 2A and 2B, figure-eight schematic 222 includes a first loop 224 (which may be a perfect circle, an oval shape, or some other circuitous shape, for instance) that represents aspects of pulmonary circulation of the patient. Figure-eight schematic 222 also includes a second loop 226 (which may be a perfect circle or an oval shape, for instance) that represents aspects of systemic circulation of the patient. In other words, top loop 224 represents circulation in the lungs of the patient and bottom loop 226 represents circulation elsewhere in the patient. Just as the lungs and the rest of a circulation system are connected by the heart, figure-eight schematic also includes a central object 228 (which may be a perfect circle or an oval shape, for instance) that represents a heart of the patient. In various embodiments, one or both of the first loop 224 and second loop 226 may include (e.g., be logically divided into) multiple arc fragments. This is best seen in Fig.2B, although the fragments are also labelled in Fig. 2A. Each arc fragment may be shaped to convey one or more measured physiological parameters of the patient, for example, various pressures and/or resistances measured within the patient. Various arrows are provided in Fig. 2A to demonstrate one example of how these various physiological parameters may be conveyed. In order that different measured physiological parameters having different values can be viewed together on a single figure-eight schematic, in various implementations, one or more measured physiological values may be normalized with respect to other measured physiological value(s). This allows for relative thicknesses of arc fragments to effectively convey a patient's hemodynamic state in an intuitive and informative manner.

In some examples, central object 228 is shaped (e.g., sized) to convey an end diastolic volume (EDV) of the patient. Cardiac output of the patient may be conveyed in some embodiments by an arc length 232 of an arc fragment 230 (referred to as "CO arc fragment" herein) of second loop 226 that extends from central object 228. Although not depicted in Figs. 2A-B, in some embodiments, a stroke volume of the patient may be conveyed by a length of a secondary arc-shaped element that extends from within CO arc fragment 230. Just downstream from CO arc fragment 230 is a mean arterial pressure (MAP) arc fragment 234. MAP arc fragment 234 may have a thickness (indicated by the double-headed arrow 235) that is selected based on a MAP measure of the patient. As an example, for a patient with high blood pressure, MAP arc fragment 234 may be relatively thick. For a patient with low blood pressure, MAP arc fragment 234 may be relatively thin. The thickness of CO arc fragment 230 conforms to (e.g., may be the same as) the MAP encoding arc 234. Rendered just downstream from MAP arc fragment 234 is an arc-shaped envelope 236 with an arc-shaped channel 238. Arc-shaped channel 238 may be bored or have a bore that is sized to convey systematic vascular resistance (SVR) by constricting a thickness of a portion of second loop 226, as shown by the inwardly pointing arrows 239 at bottom right in Fig. 2A. As an example, for a patient with high SVR arc fragment 238 may be relatively thin. For a patient with low SVR arc fragment 238 may be relatively thick. Downstream from arc-shaped envelop 236, a central venous pressure (CVP) arc fragment 240 may be shaped to convey a CVP measurement of the patient, which also may a reasonable approximation of right atrial pressure (RAP) and/or as a surrogate for preload. After CVP arc fragment 240, flow returns to central object 228, which as noted above corresponds to the heart of the patient. Thus, for instance, a thickness of CVP arc fragment 240 may represent a pressure of venous blood returning to the heart of the patient. Attention will now turn to first loop 224 representing pulmonary circulation of the patient. A mean pulmonary arterial pressure (MPAP) arc fragment 241 may be shaped to have a width (conveyed by the double-headed arrow 243) that conveys a MPAP of the patient. Similarly, a pulmonary artery wedge pressure (PAWP) or pulmonary artery occlusion pressure (PAOP) arc fragment 242 may be shaped to have a width (conveyed by the double-headed arrow 245) that conveys a PAWP/PAOP measurement of the patient. Similarly to arc-shaped envelope 236 in second loop 226 (systemic circulation) of the patient, another arc-shaped envelope 244 with an arc-shaped channel 246 may be provided between MPAP arc fragment 241 and PAWP arc fragment 242. Arc-shaped channel 246 may be bored or have a bore that is sized over a portion of first loop 224 to convey pulmonary vascular resistance (PVR) by constricting its thickness, as shown by the inwardly pointing arrows 247 at top left in Fig. 2A. For example, for a patient with high PVR arc fragment 246 may be relatively thin.

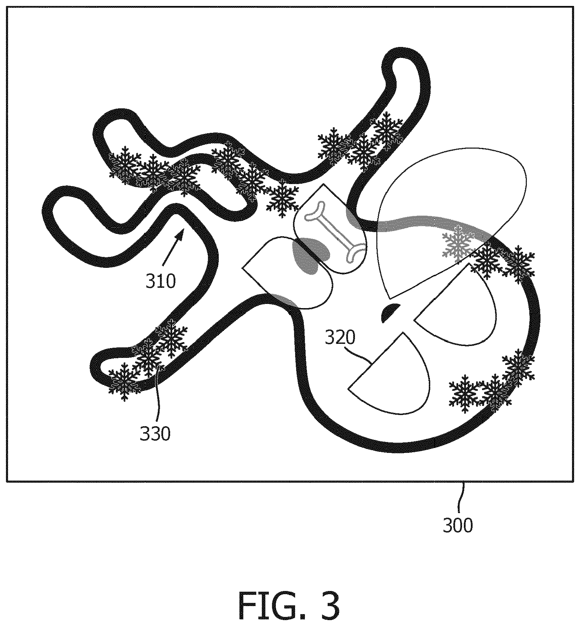

Fig. 3 shows another visualization of a physiological state of a patient in the form of an avatar 300 which changes its appearance to reflect measured values of physiological parameters of the patient. This avatar 300 has been described in references [1], [2], both of which are hereby incorporated by reference in as far as pertaining to the avatar and to the adaptation of the avatar to reflect measured values of physiological parameters of the patient. As described in [1] and [2], the avatar may be comprised of a number of visual elements which may be adapted to show physiological information of the patient. For example, one or more visual elements representing the skin of the avatar 300 may be adapted in terms of color to show oxygen saturation, for example by changing the skin to purple to represent low oxygen saturation. Another example is that one or more visual elements representing the eyes of the avatar 300 may be adapted to show high brain activity by changing their shape to represent open eyes. Yet other example is that visual elements may assume the shape of heatwaves to show a high body temperature.

In the particular example of Fig. 3, the avatar 300 is shown to have been adapted to visually represent the status of a patient with low neuromuscular activity, which can be seen from the visual elements 310 representing the legs being adapted to show the legs as floppy, i.e., hanging limply. In addition, the avatar 300 is adapted to show the patient having low brain activity, which can be seen from the visual elements 320 representing the eyes showing the eyes as closed. In addition, the avatar 300 is adapted to show the patient having a low core body temperature from visual elements 330 representing snowflakes.

Figs. 2A, 2B and Fig. 3 each show different examples of a patient status view but have in common that the patient status view does not allow for an additional temporal axis as the spatial dimensions in each view are already used to show spatial information (e.g., a spatial representation of a patient or a spatial representation of patient's circulatory system). In such patient status views, there may be no space to add a temporal axis.

Fig. 4 shows a longitudinal visualization 400 of measurement data of a patient, which visualization is elsewhere also referred to as trend view. The trend view 400 is shown to visualize the measured values of various physiological parameters, e.g., heart rate (HR), cardiac index (CI), stroke volume index (SVI), mean arterial pressure (MAP) and systolic blood pressure (SBP). In particular, the measured values are for each physiological parameter shown as the y-values of a respective graph 440, with the x-values of each of the graphs representing a common timeline 410. Compared to the patient status view shown in Figs. 2A-3, the longitudinal visualization 400 does include a temporal axis, but at the expense of being able to visualize other types of data along the x-axis. Also shown is a visual element 420, which element is further explained with reference to Figs. 6-9.

Fig. 5 shows another type of longitudinal visualization 402 of measurement data, with this visualization being similar to that of Fig. 4 but, instead of showing the measurement values of the physiological parameters as y-values of a graph, the measurement values are classified in a limited number of categories (e.g., normal, abnormal, critically abnormal) which categories may be visualized by color or pattern 442. Such a visualization is more compact along the vertical axis, allowing for more physiological parameters to be visualized in the same or similar space as the visualization 400 of Fig. 4.

Fig. 6 shows an example of a graphical output of the system 100 of Fig. 1, showing (part of a) graphical user interface which simultaneously shows a visualization 500 of a physiological state of a patient, e.g., a patient status view 500, and a longitudinal visualization 510 of measurement data of the patient, e.g., a trend view 510. The patient status view 500 may in this example be an embodiment of the figure-eight schematic which was earlier discussed with reference to Figs. 2A, 2B and may represent a circulatory system of the patient, while the trend view 510 may in this example be the type of trend view shown in Fig. 5. When comparing the physiological parameters shown in the trend view 510 to those visualized (by way of width, length, etc. of visual elements) and additionally numerically displayed in the patient status view 500, one can see that some physiological parameters overlap, e.g., the HR, CI, SVI and SBP, while the patient status view 500 may additionally visualize and numerically show other types of physiological parameters, including other advanced hemodynamic parameters, while the trend view 510 may show the mean arterial pressure (MAP) not shown in the patient status view 500. In general, as also described elsewhere, the sets of physiological parameters in each view 500, 510 may be the same but also different. For example, one set may be a subset of the other set, or there may be an overlap between both sets, or one set may have been derived from the other, etc.

Fig. 6 illustrates further functionality of the graphical user interface provided by the system 100 of Fig. 1, in that the graphical user interface may dynamically link the patient status view 500 and the trend view 510, in that a user may select a time instance in the longitudinal visualization 510, causing the visualization 500 of the physiological state of the patient to adapt to the measured values of the physiological parameters at the selected time instance. In the example of Fig. 6, the user may slide a visual element 520 (a 'slider') along the time axis to select the time instance. In particular, the slider may be a vertical line that indicates the time, and which may be dragged towards the left or right. As a result, a time instance may be selected (e.g., 13:55) and the patient status view 500 may be adapted to the measured values of the physiological parameters at that particular time instance. As shown in Fig. 6, at that select time instance, i.e., at 13:55, the pulmonary vascular resistance index (PVRI) may have been 240, the pulmonary artery pressure (PAP) may have been 16, the extravascular lung water index (ELWI) may have been 6, etc. It will be appreciated that to be able to adapt the patient status view 500 to the measured values at 13:55 or at any other past time instance, the system 100 may temporarily buffer the measured values, e.g., on an internal data storage, or if such measured values are stored elsewhere, e.g., on an external data storage, access the measured values at the selected time instance.

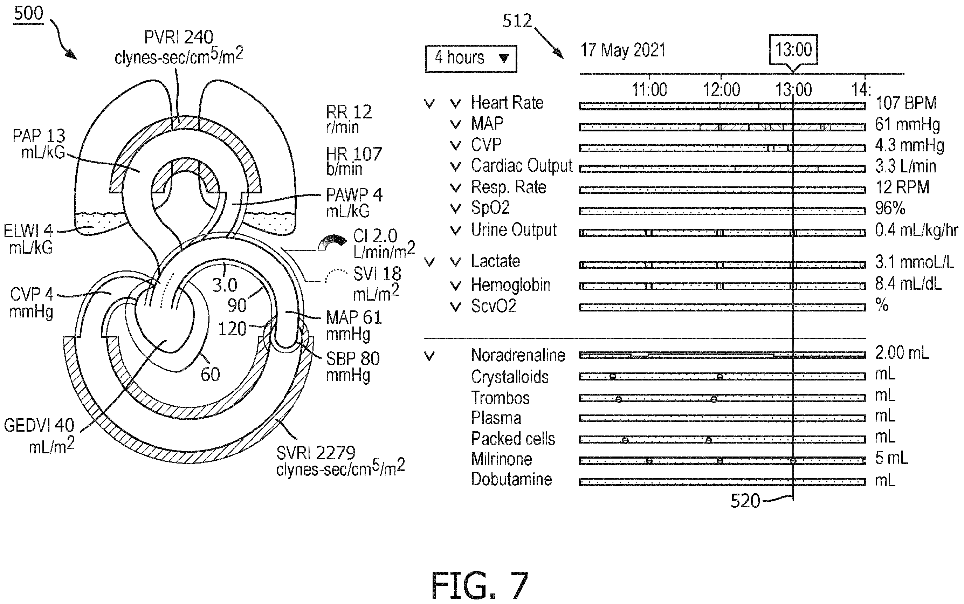

Fig. 7 is similar to Fig. 6 but shows another type of longitudinal visualization 512, namely the type previously shown in Fig. 5, while otherwise resembling Fig. 6.

It will be appreciated that, in general, in the trend view, the physiological parameters may be grouped, which may cause the physiological parameters to be shown adjacently to each other in accordance with a group. Such grouping may be done automatically, e.g., based on predefined rules, but may also be user definable. For example, a user may select or drag-and-drop graphs of physiological parameters in the trend view, which could cause the selected or dragged-and-dropped graphs to be grouped and placed together. Another example is that a user may select certain physiological parameters in the patient status view, with the selected physiological parameters then being grouped as a group in the trend view. In general, the grouping may have a clinical relevance. For example, physiological parameters in the trend view may be grouped by preload (tank), contractility (pump) and afterload (pipes). This grouping may allow a clinician to better understand and identify where the hemodynamic problem is and where to direct to intervention. Two additional groups for core parameters (general) and oxygenation may be added. Alternatively, physiological parameters in the trend view may be grouped by pulmonary circulation and systemic circulation. This grouping may be especially relevant for clinicians when a pulmonary artery catheter is placed on the patient. Additional groups for core parameters (general) and oxygenation may be added. Alternatively, physiological parameters in the trend view may be grouped by flow/volumes related parameters, pressures, fluids as vasculature. An additional group for respiration may be added. In some examples, a grouping in the trend view may cause measurement values of physiological parameters which may be displayed in the patient status view to be grouped accordingly.

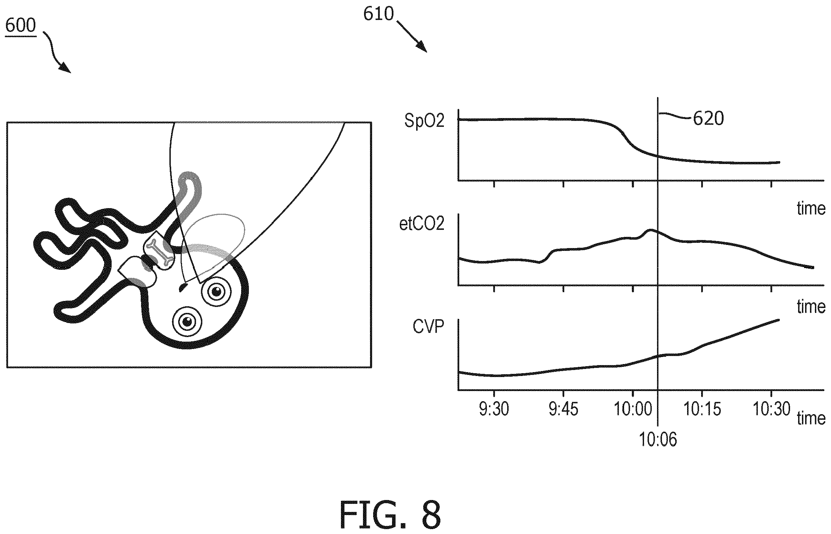

Fig. 8 is similar to Fig. 6 but shows another type of visualization of the physiological state of the patient, namely an avatar of the type as shown in Fig. 3, and another type of longitudinal visualization 610 which, like the longitudinal visualization 400, 500 of Figs. 4 and 6, shows the physiological parameters by setting out their measurement values against the y-axis of a graph, with the x-axis representing a common timeline. In addition, like the longitudinal visualization 400, 500 of Figs. 4 and 6, the user is enabled to select a time instance on the common timeline by dragging a slider 620 left and right.

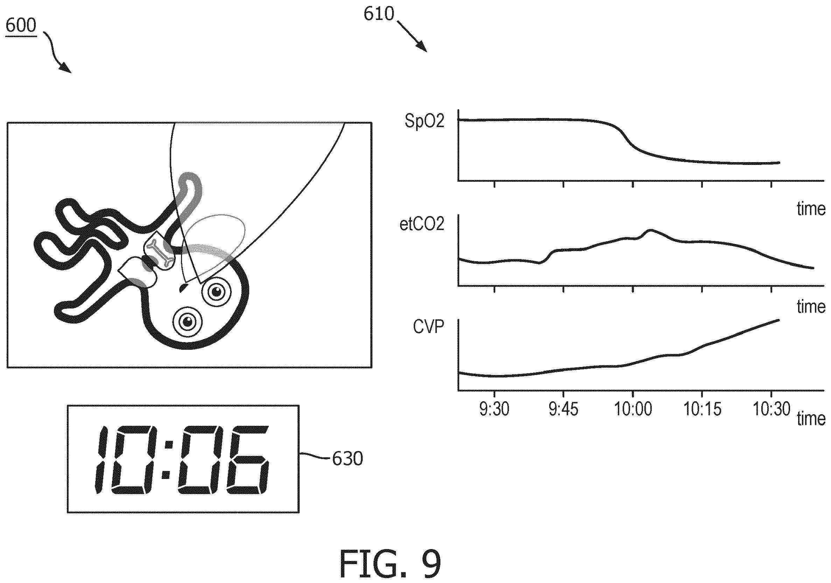

Fig. 9 is similar to Fig. 8 and shows another way of selecting a time instance. Namely, instead of using a slider, there may be a numerical input field 630 in which the user may enter the time, e.g., in a clock-like format by specifying an hour and minute.

It will be appreciated that various other types of patient status views and trend views may be shown as well. For example, instead of the figure-eight schematic and the avatar previously described, also a Frank-Starling curve may be used to visualize the physiological state of a patient. In such an example, the trend view may preferably include the stroke volume and left ventricular end-diastolic pressure as a function of time and may include other parameters related to the heart condition, such as heart rate. By selecting a time instance in the trend view, the Frank-Starling curve may be updated to reflect the measurement values of the physiological parameters at the selected time instance.

In some examples, the patient status view may be sequentially adapted, and thereby effectively animated, at a number of time instances until or from the selected time instance onwards. In such examples, the selected time instance may effectively function as a starting time or end time of the animation of the patient status view. In some examples, the user may select both a starting time and an end time, e.g., using two sliders, by sequentially dragging one slider, by numerically entering a start time and end time, etc., and the system may animate the patient status view within the time window defined by the starting time and the end time. In some examples, the patient status view 500 may be a frame from a video, for example a frame from an ultrasound video, and the video may be played-back starting from the selected time instance or until the selected time instance. In some examples, the user may control the playback of the animation or video, e.g., using playback controls (which may also be referred to as 'presentation controls'). Such playback controls may for example comprise functions such as a pause function, a resume playback function, a forward function, a reverse function, a loop selection function, and a playback speed adjustment function. Other examples of playback controls include, but are not limited to adjustments in number of visual elements (e.g., detail level of an avatar), display-related visualization options (e.g., 2D, 3D, virtual reality, augmented reality), which playback device or display to use, etc. In general, the animation or video may be played back faster than real-time, e.g., 2x, 4x, 8x, 16x, 32x, 64x, ... as fast, but also slower, e.g., 0.5x, 0.25x, etc. The playback may be forward in time, e.g., from a past time instance towards the present, but also backward in time, e.g., towards the past.

It will be appreciated that the patient status view and the trend view may also be dynamically linked in other ways besides the selection of a time instance. For example, alarm limits (also referred to as 'alarm thresholds') or target values for physiological parameters of a particular patient may be set by user input. For example, a user may, in a figure-eight schematic of the type shown in Figs. 2A, 2B, 6, 7, set the alarm limit or target value by specifying a desired width, length, orientation, etc. of a visual element which relates to a particular physiological parameter. The alarm limit or target value may then also be visualized by the system in the trend view, for example as a line or tick mark on the y-axis of the graph of the physiological parameter in the trend view. Additionally, or alternatively, alarm limits or target values may also be set by user input in the trend view, e.g., by the user being able to select a value on the vertical axis of the graph of a particular physiological parameter, with the selected alarm limit or target value then being visualized in the patient status view as a marker indicating the desired width, length, orientation, etc. of the visual element which represents the particular physiological parameter in the patient status view.

Yet another example of the dynamic linking of the patient status view and the trend view may be the following: a clinician may wish to save a particular view in the patient status view. By a simple click on the patient status view, a time stamp might be set, which may also be visible as a line or tick mark on the horizontal (time) axis/axes in the trend view. In other words, the patient status view at a particular time instance may be bookmarked by a user, with a visualization of the bookmark being presented in the trend view in visual relation to the time instance on the common timeline (e.g., as the aforementioned line or tick mark). In general, while Figs. 6-9 show the patient status view at the left-hand side of the display and the trend view at the right-hand side of the display, various other simultaneous arrangements of both types of views are possible as well, e.g., the patient status view at the right and the trend view at the left, both views above each other, etc.

In general, while the system and method for patient monitoring has been described for monitoring and visualizing measured values of physiological parameters, the same type of visualizations as for example are shown in Figs. 6-9 may be generated for a simulated patient. For example, a so-called 'digital twin' may be used, which may be a simulation model of the patient which may allow virtual measurements. While the simulation model may be based on actual measured physiological values of a patient, such virtual measurements may allow the selection if time instances in the future, e.g., to estimate changes in the patient's condition in the future, or to select time instances in the past, e.g., where actual measurement values were not available, are missing or have been corrupted.

In general, while the trend view has been described as having a (semi-) continuous timeline which is specified in hours, minutes, etc., the timeline may also be constituted by events which follow each other in time, with each event being associated with a time instance at which the event occurred. For example, a protocol followed by a clinician may comprise a sequence of steps. Each of these steps may represent an event, with the sequence of events forming the common timeline. As such, the trend view may present measurement values for a first event, for a second event, etc. The user may be enabled to select a time instance by selecting an event, with the patient status view then being changed to visualize the physiological state of the patient at the time instance of the event.

While the aforementioned examples take place in the medical domain, the dynamically linked patient status view and trend view may also be used in other domains, such as for example in endurance sports. Here, relevant physiological measurements include VO2max, resting heart rate, anaerobic threshold, muscle strength, etc. while other, non-physiological, parameters might include step/stroke frequency, step/stroke length, speed, etc. The sporter and/or his/her coach may wish to see want to look at the trends of these parameters over time, and may especially be interested in their response to certain types of training, rest periods, sleep, the use of an altitude tent, supplements, etc. Next to trend lines showing the actual measurement values of physiological parameters, also a visualization of the physiological state of the sporter may be given, for example in the form of an avatar of a sporter which is adapted to the sporter's physiological condition. For example, the avatar may look less or more muscular over time (corresponding to his/her strength) or change in heart size (where a big heart could correspond to a so-called 'sports heart').

In general, the patient may be a human but also an animal patient. In general, the system and method may be used to visualize advanced hemodynamic parameters, but also other organ system-based physiological parameters such as neurological, renal, respiratory, etc.

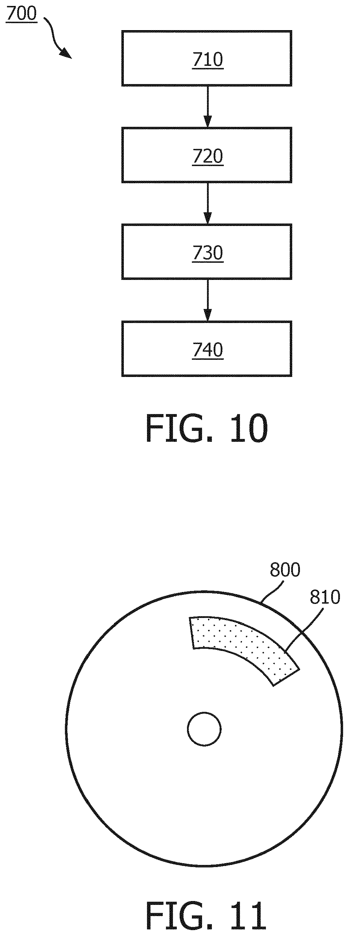

Fig. 10 shows a block-diagram of computer-implemented method 700 for patient monitoring. The method 700 may correspond to an operation of the system 100 of Fig. 1. However, this is not a limitation, in that the method 700 may also be performed using another system, apparatus or device. The method 700 is shown to comprise, in an operation titled "RECEIVING MEASUREMENT DATA", receiving 710 measurement data comprising measured values of physiological parameters of a patient at consecutive time instances, in an operation titled "PROVIDING TREND VIEW", providing 720, on a display, a trend view which provides a longitudinal visualization of the measurement data by setting out the measured values of a first set of the physiological parameters at the consecutive time instances against a common timeline, in an operation titled "PROVIDING PATIENT STATUS VIEW", providing 730, on the display, a patient status view in separation of the trend view, wherein said providing of the patient status view comprises providing a visualization of a physiological state of the patient at a select time instance, wherein the visualization is adapted to the measured values of a second set of the physiological parameters at the select time instance, and in an operation titled "DYNAMICALLY LINKING TREND VIEW AND PATIENT STATUS VIEW", dynamically linking 740 the trend view and the patient status view by receiving user input indicative of a selection of a past time instance, and in response, adapting the visualization of the physiological state to the measurements of the second set physiological parameters at the past time instance. It will be appreciated that in general, operations of method 700 of Fig. 10 may be performed in any suitable order, e.g., consecutively, simultaneously, or a combination thereof, subject to, where applicable, a particular order being necessitated, e.g., by input/output relations. Operations may also be performed as part of other operations.

The method may be implemented on a computer as a computer implemented method, as dedicated hardware, or as a combination of both. As also illustrated in Fig. 11, instructions for the computer, e.g., executable code, may be stored on a computer readable medium 800, e.g., in the form of a series 810 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 11 shows an optical disc 800.

Examples, embodiments or optional features, whether indicated as nonlimiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for patient monitoring, comprising:
- an input interface (120) for receiving measurement data (42) comprising measured values of physiological parameters (40) of a patient at consecutive time instances;
- a user interface subsystem (180) comprising:
- a user input interface (184) to a user input device (80) for receiving user input (82);
- a display output (182) to a display (60) for displaying output of the system;
- a processor subsystem (140) configured to, via the user interface subsystem:
- provide a trend view (400, 402, 510, 512) which provides a longitudinal visualization of the measurement data by setting out the measured values of a first set of the physiological parameters at the consecutive time instances against a common timeline;
- provide a patient status view (222, 500, 600) in separation of the trend view, wherein the patient status view provides a visualization of a physiological state of the patient at a select time instance, wherein the visualization of the physiological state is adapted to the measured values of a second set of the physiological parameters at the select time instance; and
- dynamically link the trend view and the patient status view by:
- receiving a selection of a past time instance;
- in response, adapting the visualization of the physiological state to the measurements of the second set physiological parameters at the past time instance.

2. The system (100) according to claim 1, wherein the visualization (500, 600) of the physiological state of the patient comprises one or more visual elements, wherein the visualization is adapted to the measured values of the second set of the physiological parameters by adjusting a visual attribute of at least one of the one or more visual elements.

3. The system (100) according to claim 2, wherein the one or more visual elements visually represent one or more anatomical or physiological attributes of the patient.

4. The system (100) according to claim 2 or 3, wherein the visual attribute is one of: a spatial attribute such as a position, orientation, size or shape, a color, a transparency, and a pattern, of the visual element.

5. The system (100) according to any one of claims 2 to 4, wherein the patient status view comprises an avatar (600) or a visual representation (222, 500) of a physiological system of the patient.

6. The system (100) according to any one of claims 1 to 5, wherein the processor subsystem (140) is configured to, via the user interface subsystem (180), enable a user to provide the user input indicative of the selection of the past time instance, for example by specifying a point on the common timeline.

7. The system (100) according to claim 6, wherein the processor subsystem (140) is configured to enable the user to specify the point on the common timeline by at least one of:
- moving a visual element onscreen relative to a visualization of the common timeline in the trend view;
- entering a numerical value, and
- selecting a visual element which modifies a currently selected time instance.

8. The system (100) according to any one of claims 1 to 7, wherein the processor subsystem (140) is configured to, via the user interface subsystem (180), animate the visualization of the physiological state of the patient shown in the patient status view by sequentially adapting the visualization of the physiological state to the measured values of the second set of the physiological parameters at the consecutive time instances and by using the selected time instance as start-time or end-time of said animation.

9. The system (100) according to claim 8, wherein the processor subsystem (140) is configured to provide, via the user interface subsystem (180), playback controls to enable the user to control said animation of the visualization of the physiological state, wherein the playback controls comprise at least one of: a pause function, a resume playback function, a forward function, a reverse function, a loop selection function, and a playback speed adjustment function.

10. The system (100) according to claim 8 or 9, wherein the processor subsystem (140) is configured to enable, via the user interface subsystem (180), the user to select a physiological parameter in the trend view to, in the patient status view, enable or disable adapting the visualization of the physiological state to the select physiological parameter, or to highlight said adaptation in the visualization of the physiological state.

11. The system (100) according to any one of claims 1 to 10, wherein the processor subsystem (140) is configured to enable, via the user interface subsystem (180), a user to set an alarm threshold for at least one of the physiological parameters in one of: the trend view (400, 402, 510, 512) and the patient status view (222, 500, 600), wherein the processor subsystem is configured to visualize the alarm threshold in the other one of: the trend view and the patient status view.

12. The system (100) according to any one of claims 1 to 11, wherein the first set and the second set of the physiological parameters are identical or different by one of said sets being a subset of, or being derived from, the other set of the physiological parameters.

13. The system (100) according to any one of claims 1 to 12, wherein the physiological parameters comprise one or more of: cardiac output (CO), cardiac index (CI), stroke volume (SV), stroke volume index (SVI), stroke volume variation (SVV), ejection fraction (EF), systemic vascular resistance (SVR), systemic vascular resistance index (SVRI), pulmonary vascular resistance (PVR), pulmonary vascular resistance index (PVRI), pulmonary artery pressure (PAP), pulmonary artery wedge pressure (PAWP), extravascular lung water index (ELWI) and global end-diastolic volume index (GEDVI).

14. A computer-implemented method (700) for patient monitoring, comprising:
- receiving (710) measurement data comprising measured values of physiological parameters of a patient at consecutive time instances;
- on a display, providing (720) a trend view which provides a longitudinal visualization of the measurement data by setting out the measured values of a first set of the physiological parameters at the consecutive time instances against a common timeline;
- on the display, providing (730) a patient status view in separation of the trend view, wherein said providing of the patient status view comprises providing a visualization of a physiological state of the patient at a select time instance, wherein the visualization is adapted to the measured values of a second set of the physiological parameters at the select time instance; and
- dynamically linking (740) the trend view and the patient status view by:
- receiving a selection of a past time instance;
- in response, adapting the visualization of the physiological state to the measurements of the second set physiological parameters at the past time instance.

15. A transitory or non-transitory computer-readable medium (800) comprising data (810) representing a computer program, the computer program comprising instructions for causing a processor system to perform the method according to claim 14.
